(19) **Europäisches Patentamt / European Patent Office / Office européen des brevets**

(11) **EP 2 508 256 A1**

(12) # EUROPEAN PATENT APPLICATION
published in accordance with Art. 153(4) EPC

(43) Date of publication:
**10.10.2012 Bulletin 2012/41**

(21) Application number: **10833373.3**

(22) Date of filing: **29.11.2010**

(51) Int Cl.:
$B01J\ 27/24^{(2006.01)}$     $B01J\ 37/10^{(2006.01)}$
$C07C\ 51/235^{(2006.01)}$     $C07C\ 57/055^{(2006.01)}$
$C07B\ 61/00^{(2006.01)}$     $C07C\ 51/377^{(2006.01)}$
$C07C\ 57/05^{(2006.01)}$

(86) International application number:
**PCT/JP2010/071252**

(87) International publication number:
**WO 2011/065529 (03.06.2011 Gazette 2011/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2009 JP 2009271884**

(71) Applicant: **Nipponkayaku Kabushikikaisha Tokyo 102-8172 (JP)**

(72) Inventors:
• **SUDO, Atsushi**
  **Takasaki-shi**
  **Gunma (JP)**
• **KURAKAMI, Tatsuhiko**
  **Sanyoonida-shi**
  **Yamaguchi (JP)**

• **ARATANI, Yasuhiro**
  **Tokyo 102-8172 (JP)**
• **NOWATARI, Hiroyoshi**
  **Takasaki-shi**
  **Gunma (JP)**
• **SAKAI, Fumio**
  **Sanyoonida-shi**
  **Yamaguchi (JP)**
• **KOBAYASHI, Tomoaki**
  **Sanyoonida-shi**
  **Yamaguchi (JP)**

(74) Representative: **Gille Hrabal**
  **Patentanwälte**
  **Brucknerstrasse 20**
  **40593 Düsseldorf (DE)**

(54) **PROCESS FOR PRODUCTION OF CATALYST FOR USE IN PRODUCTION OF METHACRYLIC ACID, AND PROCESS FOR PRODUCTION OF METHACRYLIC ACID**

(57)     An object of the present invention is to provide a process for stably producing a catalyst for methacrylic acid production exhibiting high activity and high performance.

The process for producing a catalyst for methacrylic acid production according to the present invention is a process for producing a catalyst comprising, as an active component, a hetero polyacid salt essentially containing molybdenum, phosphorus, vanadium and copper and one or more ingredients selected from alkali metals, alkaline earth metals and ammonia, the process comprising:

(A) a step of drying a slurry obtained by mixing a compound containing these essential ingredients and water, thereby obtaining a dry powder;

(B) a pre-baking step of baking the dry powder in a baking apparatus into which a gas having a controlled absolute humidity has been introduced, thereby obtaining a catalyst ingredient powder;

(C) a catalyst molding step of molding the catalyst ingredient powder under an atmosphere gas having a controlled absolute humidity; and

(D) a baking step of baking the molded catalyst in a baking apparatus into which a gas having a controlled absolute humidity has been introduced,

the gas to be introduced in the pre-baking step of the above (B) having an absolute humidity of 0.007 to 0.025 kg/kgDA;

the atmosphere gas in the catalyst molding step of the above (C) having an absolute humidity of 0.007 to 0.025 kg/kgDA; and

the gas to be introduced in the baking step of the above (D) having an absolute humidity of 0.007 to 0.025 kg/kgDA.

EP 2 508 256 A1

**Description**

Technical Field

The present invention relates to a process for producing a catalyst for methacrylic acid production for use in the production of methacrylic acid by vapor-phase catalytic oxidation of methacrolein, isobutyraldehyde, isobutyric acid, or the like.

Background Art

As catalysts to be used for producing methacrylic acid by vapor-phase catalytic oxidation of methacrolein, isobutyraldehyde, isobutyric acid, or the like, it is known that those having a structure of a hetero polyacid and/or a salt thereof are effective, and there are a large number of proposals regarding compositions, physical properties, and production processes thereof.

These are usually produced by steps of preparation of a raw material mixed solution, drying, molding, baking, and the like. With regard to the production of the catalysts stably exhibiting high performance, there are a large number of reports on molding methods and baking methods.

For example, in Patent Document 1, a process of molding with mixing heat-resistant fibers has been proposed for the purpose of enhancing the strength of the catalysts. Moreover, in Patent document 2, there has been proposed a process of crying a slurry containing essential ingredients and subsequently baking the resulting dry powder before molding for the purpose of improving catalyst moldability. In Patent document 3, there has been proposed a process of treating a molded catalyst under a certain humidity atmosphere and subsequently performing baking for the purpose of producing methacrylic acid in high yields.

Furthermore, the present applicant has proposed a process for producing a hetero polyacid catalyst stably exhibiting high activity and high performance even in the case where the catalyst is molded by a coating method in Japanese Patent Application No. 2009-126943 (filed on May 26, 2009 (Patent Document 4)). The production process is characterized in that water content of a catalyst for use in molding, temperature and humidity in the molding step, and humidity in the baking step are controlled whey a catalyst containing a hetero polyacid as an active component is molded by a coating method and baked.

Related Art

Patent Document

Patent Document 1: JP-B-2-36296
Patent Document 2: JP-A-2006-314923
Patent Document 3: Japanese Patent No. 3797148
Patent Document 4: Japanese Patent Application No. 2009-126943 (filed on May 26, 2009)

Summary of Invention

Problems to Be Solved by the Invention

However, in a catalyst comprising a hetero polyacid salt as an active component, a process of performing baking simply after a molded catalyst has been treated under a certain humidity atmosphere alone as described in Patent Document 3 was insufficient for stable production of a high-performance catalyst. Particularly, there often occurred a case where intrinsic catalytic performance was not exhibited depending on atmosphere conditions in a step of baking a dry powder, a molding step, and a step of drying and baking the molded article. Accordingly, it was found that it was extremely difficult to produce a catalyst stably exhibiting high activity and high performance as an industrial catalyst for methacrylic acid production.

An object of the present invention is to provide a process for producing a catalyst for methacrylic acid production stably exhibiting high activity and high performance, with overcoming these difficulties.

Means for Solving the Problems

As a result of extensive studies, the present inventors have found that, in a hetero polyacid salt containing molybdenum, phosphorus, vanadium and copper and one or more ingredients selected from alkali metals, alkaline earth metals and ammonia as essential ingredients, it is possible to produce a catalyst for methacrylic acid production stably exhibiting high activity and high performance by controlling atmosphere humidity and temperature in a step of baking a dry powder before molding, a step of molding the dry powder, and a step of baking the molded article. Thus, they have accomplished the present invention.

Namely, the present invention relates to:

(1) a process for producing a catalyst comprising, as an active component, a hetero polyacid salt essentially containing molybdenum, phosphorus, vanadium and copper and one or more ingredients selected from alkali metals, alkaline earth metals and ammonia, the process comprising:

(A) a step of drying a slurry obtained by mixing compound(s) containing these essential ingredients and water, thereby obtaining a dry powder;
(B) a pre-baking step of baking the dry powder in a baking apparatus into which a gas having a controlled absolute humidity has been introduced, thereby obtaining a catalyst ingredient powder;
(C) a catalyst molding step of molding the catalyst ingredient powder under an atmosphere gas having a controlled absolute humidity; and
(D) a baking step of baking the molded catalyst in a baking apparatus into which a gas having a controlled absolute humidity has been introduced,

the gas to be introduced in the pre-baking step of the above (B) having an absolute humidity of 0.007 to 0.025 kg/kgDA;
the atmosphere gas in the catalyst molding step of the above (C) having an absolute humidity of 0.007 to 0.025 kg/kgDA; and
the gas to be introduced in the baking step of the above (D) having an absolute humidity of 0.007 to 0.025 kg/kgDA;

(2) The process according to the above (1), wherein the essential active ingredients of the hetero polyacid further include antimony.

(3) The process according to the abode (2), which is a for producing a catalyst comprising, as an active component, a hetero polyacid salt essentially containing molybdenum, phosphorus, vanadium, copper and antimony and one or more ingredients selected from alkali metals, alkaline earth metals and ammonia, the process comprising:

(A) a step of drying a slurry obtained by mixing compound(s) containing these essential ingredients except antimony and water, thereby obtaining a dry powder;
(B) a pre-baking step of baking a mixture of the dry powder and a compound containing antimony in a baking apparatus into which a gas having a controlled absolute humidity has been introduced, thereby obtaining a catalyst ingredient powder;
(C) a catalyst molding step of molding the catalyst ingredient powder under an atmosphere gas having a controlled absolute humidity; and
(D) a baking step of baking the molded catalyst in a baking apparatus into which a gas having a controlled absolute humidity has been introduced;

the gas to be introduced in the pre-baking step of the above (B) having an absolute humidity of 0.007 to 0.025 kg/kgDA;
the atmosphere gas in the catalyst molding step of the above (C) having as absolute humidity of 0.007 to 0.025 kg/kgDA; and
the gas to gas introduced in the baking step of the above (D) having an absolute humidity of 0.007 to 0.025 kg/kgDA.

(4) The process according to the above (2), which is a process for producing a catalyst comprising, as an active component, a hetero polyacid salt essentially containing molybdenum, phosphorus, vanadium, copper and antimony and one or more ingredients selected from alkali metals, alkaline earth metals and ammonia, the process comprising:

(A) a step of drying a slurry obtained by mixing compound(s) containing these essential ingredients except antimony and water, thereby obtaining a dry powder;

(B) a pre-baking step of baking the dry powder in a baking apparatus into which a gas having a controlled absolute humidity has been introduced, thereby obtaining a catalyst ingredient powder;
(C) a catalyst molding step of molding a mixture of the catalyst ingredient powder and a compound containing antimony under an atmosphere gas having a controller absolute humidity; and
(D) a baking step of baking the molded catalyst in a baking apparatus in which a gas having a controlled absolute humidity has been introduced;

the gas to be introduced in the pre-baking step of the above (B) having an absolute humidity of 0.007 to 0.025 kg/kgDA;
the atmosphere gas in the catalyst molding step of the above (C) having an absolute humidity of 0.007 to 0.025 kg/kgDA; and
the gas to be introduced in the baking step of the above (D) having an absolute humidity of 0.007 to 0.025 kg/kgDA.
(5) The process according to any one of the above (1) to (4), wherein the gas to be introduced in the pre-baking step of the above (B), the atmosphere gas in the catalyst molding step of the above (C), and the gas to be introduced in the baking step of the above (D) are gases having a controlled temperature together with a controlled absolute humidity,

the gas to be introduced in the pre-baking step of the above (B) having a temperature of 15 to 90°C and an absolute humidity of 0.007 to 0.025 kg/kgDA;
the atmosphere gas in the catalyst step of the above (C) haying a temperature of 15 to 35°C and an absolute humidity of 0.007 to 0.025 kg/kgDA; and
the gas to be introduced in the baking step of the above (D) having a temperature of 15 to 90°C and an absolute humidity of 0.007 to 0.025 kg/kgDA.
(6) The process according to any one of the above (1) to (5), the active ingredients of the hetero polyacid salt are molybdenum, phosphorus, vanadium, copper and an alkali metal.

(7) The process according to any one of the above (1) to (5), wherein the active ingredients of the hetero polyacid salt are molybdenum, phosphorus, vanadium, copper, an alkali metal and ammonia.

(8) The process according to any one of the above (1) to (7), wherein the alkali metal is cesium.

(9) The process according to any one of the above (1) to (8), wherein the baking temperature is 200 to 400°C in the pre-baking step of the above (B).

(10) The process according to any one of the above (1) to (9), wherein the molding step of the above (C) is a step of coating an inactive support using a binder to form a coated catalyst.

(11) The process according to the above (10), wherein the binder is water and/or at least one liquid selected from the group consisting of organic compounds having a boiling point of 150°C or lower at 1 atm.

(12) The process according to any one of the above (1) to (11), wherein the molded article is baked at 100 to 450°C in the baking step of the above (D).

(13) A process for producing methacrylic acid by vapor-phase catalytic oxidation of methacrolein, isobutyraldehyde or isobutyric acid using the catalyst according to any one of the above (1) to (12).

Effects of the Invention

According to the production process of the present invention, a high-activity and high-performance for methacrylic acid production comprising, as an active component, a hetero polyacid salt containing molybdenum, phosphorus, vanadium and copper and one or more ingredients selected from alkali metals, alkaline earth metals and ammonia can be stably produced.

Embodiments for in Out the Invention

In the production process of the present an aqueous solution containing compound(s) containing catalyst active ingredients (molybdenum, phosphorus, vanadium and and one or more ingredients selected from alkali metals, alkaline earth metals and ammonia; hereinafter referred to as essential ingredients) or a water dispersion of the compound(s) (hereinafter collectively referred to as a slurry) is prepared, the slurry is dried and the dry powder is baked (hereinafter the step is referred to as pre-baking), and the baked powder is molded, followed by performing drying (drying after molding) and baking (main baking).
Moreover, in the present invention, the compound(s) containing the active ingredients at the preparation of the slurry not necessarily contains all the active ingredients and a part of the ingredients may be added at the step of the dry powder or after the pre-baking step.
Among the essential ingredients, sodium, lithium, potassium, rubidium, cesium and the like may be mentioned as the alkali metals, and magnesium, calcium, barium and the like may be mentioned as alkaline earth metals. The alkali metal, the alkaline earth metal and ammonia are used for neutralizing at least a part of protons of the hetero polyacid the elements to form a hetero polyacid salt.
The alkali metals, alkaline earth metals and ammonia may be used each singly or two or more thereof may be used in combination. In the combination two or more selected from the alkali metals are preferred and combination use of the alkali metal and ammonia is preferred. Among the alkali metals, cesium is particularly preferred and use of cesium alone or combination use of cesium and ammonia is further preferred.
In the present invention, metal elements other than the essential ingredients can be further incorporated as active ingredients. As the metal elements other than the essential ingredients, there may be mentioned one or more elements selected from the group consisting of arsenic, silver, manganese, zinc, aluminum, boron, germanium, tin, lead, titanium, zirconium, chromium, rhenium, bismuth, tungsten, iron, cobalt, nickel, cerium, thorium, antimony and the like, and antimony is preferred. A method of adding the metal elements other than the essential ingredients is not particularly limited unless local concentration distribution of the ingredients occurs. The metal element may be added any of (a) at the slurry preparation, (b) the pre-baking, and (c) the molding step after the pre-baking but, from the viewpoint of catalytic performance, (b) or (c) is preferred.
In the present invention, with regard to use ratios of active ingredient-containing compounds, as atomic ratios, the ratios are usually 0.1 or more and 6 or less, preferably 0.3 or more and 2.0 or less for vanadium, 0.5 or and 6 or less, preferably 0.7 or more and 2.0 or less for phosphorus, and usually 0 or more excluding 0 and 2.0 or less, preferably 0.05 or more and 1.0 or less, further preferably 0.1 or more and 0.6 or less for copper relative to 10 for molybdenum. Moreover, the ratio of the alkali metal is usually 0.01 or more and 4.0 or less, preferably 0.1 or and 2.0 or less. The alkali metal may be contained alone without containing the alkaline earth metal. In the case where the alkaline earth metal is contained, the ratio of the sum of the alkali and a half of the alkaline earth metal [alkali metal + 1/2×(alkaline earth metal)] is usually 0.01 or more and 4.0 or less, preferably 0.1 or more and 2.0 or less, and the ratio of ammonia (usually contained as an ammonium group) is usually 0.1 or and 10.0 or less, preferably 0.5 or more and 5.0 or less. Furthermore, the arbitrary ingredients, for antimony that is a preferable element, the ratio is usually 0.01 or more and 5 or less, preferably 0.05 or more and 2.0 or less. The kinds and use ratios of the other ingredients to be used according to needs are appropriately determined so that a catalyst exhibiting optimum performance is obtained according to use conditions of the catalyst and the like. The atomic ratio (composition) of the catalyst described in the present invention is one at a raw material charging stage and is a value excluding oxygen.
The following will explain embodiments according to the above steps.

Slurry Preparation

In the present as the compound(s) containing active ingredients (hereinafter referred to as an active ingredient-containing compound(s)) for use in catalyst preparation, chlorides, sulfates, nitrates, oxides, or the like of the ingredient can be used. When preferable compounds are more specifically exemplified, there be nitrates such as potassium or cobalt nitrate; oxides such as molybdenum oxide, vanadium pentoxide, antimony trioxide, cerium oxide, zinc oxide, or germanium oxide; acids (or salts thereof) such as orthophosphoric acid, phosphoric acid, boric acid, aluminum or 12 tungstophosphoric acid and the like.

Moreover, as a copper compound, it is preferred to use copper acetate (cuprous acetate, cupric acetate, basic copper or cupric oxide, preferably cupric acetate) oar copper oxide (cuprous oxide, cupric oxide),

Furthermore, as a cesium compound, it is preferred to use cesium acetate oar cesium and a cesium weak acid salt and, as an ammonium compound, it is preferred to use ammonium acetate or ammonium hydroxide.

These active ingredient-containing compounds may be used singly or two or more thereof may be used as a mixture.

The can be obtained by homogeneously mixing the active ingredient-containing compound(s) with water. As an addition order of the active ingredient-containing compounds at the slurry preparation, it is preferred that compounds containing molybdenum, vanadium, phosphorus and, if necessary, other metal elements are thoroughly and the alkali metal-containing compound, the alkaline earth metal-containing compound, the ammonium-containing compound and the copper-containing compound are added. In the case where the antimony-containing compound that is a preferable active ingredient at the slurry preparation is added, the compound is preferably added at the last among the essential active ingredient-containing compounds. More preferably, after a slurry containing the active ingredients other than the antimony-containing compound is obtained, a dry powder obtained by drying and the antimony-containing compound are and then pre-baking is performed or the antimony-containing compound is mixed after the powder is pre-baked.

As a temperature at the preparation of the slurry, it is preferred to heat the mixture to a temperature at which compounds containing molybdenum, phosphorus, vanadium and, if necessary, other metal can be thoroughly dissolved. Moreover, a temperature at the addition of the alkali metal-containing compound, the alkaline earth metal-containing compound, and the ammonium-containing compound is in the range of usually 0 to 35°C, preferably 10 to 30°C. By controlling the temperature at the addition to the above range, the resulting catalyst tends to have high activity. Therefore, at the addition of the alkali metal-containing compound, the alkaline earth metal-containing compound, and the anunonium-containing compound, it is preferred to the mixture to the above temperature range.

The amount of water to be used in the slurry is not particularly limited so as it is an amount with the total amount of the compounds to be used can be completely dissolved or can be homogeneously mixed but is appropriately determined in consideration of the drying method, drying conditions, and the like. Usually, based on 100 parts by mass of the total mass of the compounds for slurry preparation, about 200 to 2,000 parts by mass of water is used. The amount of water may be a large amount but when the amount is too large, there are such many demerits that an energy for the drying step increases, drying takes much time, and the like.

Drying

Then, the slurry obtained in the above is dried to form a catalyst ingredient powder. The drying method is not particularly limited so long as it is a method capable of completely drying the slurry. For example, drum drying, freeze drying, spray drying, evaporation to dryness and the like may be mentioned. Of these, in the present invention, the spray drying capable of drying from a slurry state to a powder or granules for a short period of time is preferred. The drying temperature in the spray drying varies depending on the concentration of the slurry, solution-transferring rate and the like but the outlet temperature of a drier is generally 70 to 150°C. On this occasion, drying is preferably performed so that the average particle size of the resulting slurry-dried matter becomes 30 to 700 $\mu$m. Although a suitable amount of water in the air to be introduced into the spray drier or the like varies depending on the drying conditions, in the usual cases, it is preferred to use an air containing such an amount of water that a dew point is 15°C or higher, for example, a method of feeding air humidified using a humidifier.

Pre-Baking

Moldability and shape and mechanical strength of a molded catalyst are remarkably improved by pre-baking the obtained dry powder (or a mixture of the dry powder and the antimony-containing compound). A gas to be introduced into a baking apparatus may be air or an inert gas such as nitrogen but is industrially preferably air.

In the present invention, during at least a period through which baking is performed at a temperature to be mentioned later, a gas having a controlled humidity and preferably a controlled temperature is introduced into a baking apparatus. By such an operation, the absolute humidity in the baking apparatus is controlled to an appropriate value. The baking apparatus to be used in the present invention is not particularly limited but, since it is necessary to regulate the humidity and preferably the temperature, from the viewpoint of stability and easiness of the regulation, it is preferred to use an apparatus of not an opened type but a closed type where a part of inner air is circulated or to place the baking apparatus in a chamber where the temperature and the humidity are regulated.

A humidity condition of the gas to be introduced into the baking apparatus is an absolute humidity of 0.007 to 0.025 kg/kgDA, preferably an absolute humidity of 0.007 to 0.021 kg/kgDA. In the case where the pre-baking is performed under a condition where the absolute humidity of the gas to be introduced into the baking apparatus is lower than 0.007 kg/kgDA, methacrolein conversion after high-temperature reaction treatment decreases in the catalytic oxidation reaction of methacrolein. On the other hand, in the case where the humidity exceeds 0.025 kg/kgDA, it is not preferred in view of costs required for humidity regulation.

A temperature of the gas to be introduced into the baking apparatus is preferably 15 to 90°C. When the temperature is lower than 15°C, there is a concern that dew condensation may occur. On the other hand, when the temperature exceeds 90°C, a huge apparatus becomes necessary. In the present invention, from the viewpoint of catalytic performance, the temperature and humidity conditions of the gas to be introduced into the baking apparatus is preferably a temperature of 15 to 90° C and an absolute humidity of 0.007 to 0.025 kg/kgDA and more preferably a temperature of 30 to 75°C, an absolute humidity of 0.007 to 0.021 kg/kgDA, and a relative humidity of 99% or less at the temperature range.

Moreover, the pre-baking can be performed at a temperature of 200 to 400°C but the temperature is preferably 250 to 380°C, more preferably 290 to 310°C. Even when the pre-baking is performed at a temperature lower than 200°C, influence on moldability tends to decrease and when the temperature exceeds 400°C, the catalytic performance is sometimes adversely influenced.

A time for the pre-baking is preferably between 3 and 12 hours, more preferably 5 and 10 hours. The baking for more than 12 hours is permissible but an effect comparable to the baking is difficult to obtain. With regard to the reason why moldability is improved by the pre-baking, the inventors have presumed that such a hetero polyacid salt as the catalyst generally has mostly a structure called Dawson type in the case where a slurry is only dried and is transformed into a structure called Keggin type by heating, the transformation may lead to the improvement of moldability.


Molding

Then, the obtained pre-baked granules are molded as follows but, since workability is improved, it is preferred to perform molding after mixing with a molding aid such as silica gel, diatomaceous earth, or alumina powder. The amount of the molding aid to be used is usually 1 to 30 parts by mass based on 100 parts by mass of the pre-baked granules. Moreover, it is useful for enhancing mechanical strength of the catalyst to use further inorganic fibers inactive to the catalyst ingredients, such as ceramic fibers or whiskers, as a strength enhancer according to needs. However, fibers reactive with the catalyst ingredients, such as potassium titanate whiskers or basic magnesium carbonate whiskers, are not preferred. The amount of the fibers to be used is usually 1 to 30 parts by mass based on 100 parts by mass of the pre-baked granules.

The pre-baked granules obtained as above or a mixture obtained by mixing the powder with the molding aid and the strength enhancer is molded into columnar articles, pellets, ring form ones, spherical ones or the like before use, in order to reduce pressure loss of a reactive gas. Particularly, since improvement in selectivity and removal of reaction heat can be expected, a coating method where an inactive support is coated with the pre-baked granules or the mixture to form a coated catalyst is preferred. As the coating method, a rolling granulation method to be described below is preferred. The method is a method of coating a support with the pre-baked granules or the mixture where, in an apparatus having a flat or uneven disk at the bottom of a fixed container, the support in the container is stirred through repetition of autorotation movement and orbital movement by rotating the disk at a high speed and a binder and the pre-baked granules or the mixture are added thereto.

As methods of adding the binder, methods of 1) mixing it into the pre-baked granules or the mixture beforehand, 2) adding it at the same time when the pre-baked granules or the mixture is added into the fixed container, 3) adding the binder after the pre-baked granules or the mixture is added into the fixed container, 4) adding the binder before the pre-baked granules or the mixture is added into the fixed container, 5) dividing each of the pre-baked granules or the mixture and the binder and adding all amount of them with appropriately combining 2) to 4), and the like may be arbitrarily adopted. Of these, in the method of 5), for example, it is preferred to perform the addition with regulating addition rates using an auto feeder or the like so that a prescribed amount thereof is supported on the support without attachment of the pre-baked granules or the mixture to walls of the fixed container and aggregation between the pre-baked granules or the mixture.

The binder is not particularly limited so long as it is at least one selected from the group consisting of water and organic compounds having a boiling point of 150°C or lower under 1 atm. In consideration of drying and the like after coating, organic compounds having boiling point of 150°C or lower are preferred. Specific examples of the binders other than water include alcohols having 1 to 4 carbon atoms, ethers such as ethyl ether, butyl ether and dioxane, esters such as ethyl acetate and butyl acetate, ketones such as acetone and methyl ethyl ketone and aqueous solutions thereof and particularly, ethanol is preferred.

In the case where ethanol is used as the binder, preferred is ethanol/water = 10/0 to 0/10 (mass ratio), preferable 10/0 to 1/9 (mass ratio). The amount of these binders to be used is usually 10 to 60 parts by mass, preferably 15 to 40 parts by mass based on 100 parts by mass of the dry powder.

Specific examples of the support usable in the present invention include spherical supports having a diameter of 1 to 15 mm, preferably 2.5 to 10 mm, made of silicon carbide, alumina, silica-alumina, mullite, alundum and the like. As these supports, those having a porosity of 10 to 70% are usually employed. Regarding the ratio of the support to the pre-baked granules or the mixture for coating, there are used amounts so that pre-baked granules or mixture/(pre-baked granules or mixture + support) is usually 10 to 75% by mass, preferably 15 to 60% by mass. Thus, the support is coated with the pre-baked granules or the mixture and the molded article obtained on this occasion usually has a diameter of about 3 to 15 mm.

Since the hetero polyacid salt remarkably changes its catalytic performance depending on the atmosphere humidity in the molding step, it is extremely important for stably producing high-performance catalyst to control the humidity to a prescribed range also in the molding step, Moreover, it is also preferred to control the atmosphere temperature in the molding step. Since these conditions influence the catalytic performance to a large extent even in the vicinity of ordinary temperature and in the range of usual humidity in the air, when molding is performed without controlling these conditions, stable production of the high-performance catalyst cannot be achieved.

In the present invention, the molding step is performed under an atmosphere of an absolute humidity of 0.007 to 0.025 kg/kgDA. Namely, the atmosphere condition of the apparatus and peripheral instruments for carrying out the molding step may be set at an absolute humidity of 0.007 to 0.025 kg/kgDA. At this time, the atmosphere temperature is not limited so long as it is a usual molding temperature but is preferably maintained in a range of 15 to 35°C. This is because occurrence of dew condensation by temperature change and occurrence of molding defects owing to attachment to the apparatus are prevented. From the viewpoint of workability and costs required for temperature and humidity adjustment, a temperature of 20 to 30°C is preferred. Moreover, since there is a concern that dew condensation on the apparatus surface and molded article surface may occur by slight temperature change when relative humidity in the air increases, an absolute humidity of 0.007 to 0.021 kg/kgDA and relative humidity of 99% or less at a temperature of 20 to 30°C are preferred.

In the case where molding is performed by the method of the present invention, since interaction between the molded article and air atmosphere depends on the amount of water contained in the air, a suitable humidity range should be controlled by not relative humidity that is commonly used but absolute humidity. As a matter of course, the absolute humidity can be converted into the relative humidity but, since saturated vapor pressure increases with temperature, the relative humidity decreases with the elevation of temperature even when the absolute humidity is the same. The absolute humidity described in the present invention is, relative to 1 kg of dry air weight excluding water vapor in the atmosphere, weight (kg) of the excluded water vapor.

When the absolute humidity of the apparatus and peripheral instruments for carrying out the molding step is lower than the aforementioned range, the conversion of methacrolein, isobutyraldehyde or isobutyric acid after high-temperature reaction treatment decreases in the catalytic oxidation reaction of methacrolein, isobutyraldehyde or isobutyric acid, so that the case is not preferred. On the other hand, when the absolute humidity is higher than the prescribed range, the powder and the molded article are prone to attach in the apparatus and there tends to occur inconveniences such as a decrease in supported amount on the support and a decrease in productivity, an increase in the amount of the catalyst powder used, and facility breakdown, so that the case is not preferred.

The temperature control in the molding step can be performed by a usual temperature-controlling instrument and, for the humidity control, a usual humidifier or dehumidifier can be used but it is necessary to have a sufficient capacity in comparison to the chamber volume of the apparatus and peripheral instruments for carrying out the molding step.

Drying after Molding and Baking

The aforementioned molded article may be charged into the baking apparatus as it is and the temperature may be elevated but, since there is a concern that the peripheral atmosphere becomes conditions of a range of explosion, it is preferred to vaporize the binder such as an alcohol by a drying step. Moreover, in order to prevent rapid vaporization of the binder, it is preferred to perform drying under an atmosphere of lower than 40°C, an absolute humidity of 0.007 to 0.021 kg/kgDA and a relative humidity of 99% or less at the temperature range as drying conditions and it is also possible to send air or an inert gas such as nitrogen for drying. The drying chamber or drying apparatus to be used in the present invention is not particularly limited but, since it is necessary to control humidity in addition to temperature, an apparatus of not an opened type but a closed type where a part of inner air is circulated is preferred from the viewpoint of stability and easiness of the regulation. Moreover, although baking may be performed in another apparatus after drying, it is convenient to use the same apparatus for baking and drying since the transfer of the molded article is not necessary after drying. A drying time is usually 0.5 to 10 hours.

When the molded article dried in the above step is baked, the baking is usually performed under an air atmosphere but, after baking under an inert gas atmosphere such as nitrogen, the baking may be further performed under an air atmosphere according to needs.

In the present invention, an atmosphere gas to be introduced into the baking apparatus is controlled so that an absolute humidity becomes 0.007 to 0.025 kg/kgDA. By such an operation, the absolute humidity in the baking apparatus is controlled to an appropriate value. The atmosphere gas may be introduced into the baking apparatus during the baking period of time but it is preferred to continuously introduce the gas from the drying period before baking. Furthermore, the atmosphere gas is desirably controlled to a temperature of 15 to 90°C. When the temperature is lower than 15°C, there is a concern that dew condensation may occur. On the other hand, when the temperature exceeds 90°C, a huge apparatus becomes necessary. In the case where baking is performed under a condition that the absolute humidity of the gas to be introduced into the baking apparatus is lower than 0.007 kg/kgDA, the conversion of methacrolein, isobutyraldehyde or isobutyric acid after high-temperatare reaction treatment decreases in the catalytic oxidation reaction of methacrolein, isobutyraldehyde or isobutyric acid. On the other hand, in the case where the humidity exceeds 0.025 kg/kgDA, it is not preferred in view of costs required for humidity regulation. Moreover, the temperature is elevated to a temperature at which the relative humidity becomes 10% or less within 30 minutes after the start of baking and a baking temperature is usually 100 to 450°C, preferably 270 to 420°C, while a baking time is 1 to 20 hours.

In the present invention, temperature and humidity conditions of the atmosphere gas to be introduced into the baking apparatus are preferably a temperature of 15 to 90°C and an absolute humidity of 0.007 to 0.025 kg/kgDA, more preferably a temperature of 30 to 75°C, an absolute humidity of 0.007 to 0.021 kg/kgDA, and a relative humidity of 99% or less at the temperature range.

In the case where baking and drying before baking are performed in the same apparatus, the temperature and the humidity may be the same setting values at the drying and baking.

Examples

The following will describe the present invention further specifically with reference to Examples but the present invention is not limited to the following Examples. In the following, conversion, selectivity and yield are defined as follows.

$$\text{Conversion (\%)} = (\text{Number of moles of reacted methacrolein})/(\text{Number of moles of fed methacrolein}) \times 100$$

$$\text{Selectivity (\%)} = (\text{Number of moles of formed methacrylic acid})/(\text{Number of moles of reacted methacrolein}) \times 100$$

$$\text{Yield (\%)} = (\text{Number of moles of formed methacrylic acid})/(\text{Number of moles of fed methacrolein}) \times 100$$

Example 1

1) Preparation of Catalyst

To 5680 ml of pure water were added 800 g of molybdenum trioxide, 40.43 g of vanadium pentoxide, and 73.67 g of 85% by mass orthophosphoric acid, and the whole was stirred at 92°C for 3 hours to obtain a reddish brown transparent solution. Then, the solution was cooled to 15 to 20°C and 307.9 g of a 9.1% by mass aqueous cesium hydroxide solution and 689.0 g of a 14.3% by mass aqueous ammonium acetate solution were gradually added thereto under stirring and aging was performed at 15 to 20°C for 1 hour to obtain a yellow slurry.

Subsequently, further, 709.9 g of a 6.3% by mass aqueous cupric acetate solution was gradually added to the slurry and aging was further performed at 15 to 20°C for 30 minutes. Then, the slurry was spray-dried to obtain granules.

Composition of the obtained granules is as follows:

$Mo_{10}V_{0.8}P_{1.15}Cu_{0.4}Cs_{0.3}(NH_4)_{2.3}$.

Then, 320 g of the granules was baked at 310°C over a period of 5 hours under a stream of air humidified so as to be a relative humidity of 60% (absolute humidity: 0.012 kg/kgDA) at 25°C. There was observed a weight loss of about 4% by mass by the pre-baking. Thereinto were homogeneously mixed 22.7 g of antimony trioxide and 45 g of a strength enhancer (ceramic fibers), and the mixture was coat-molded on 300 g of a spherical porous alumina support (average particle size: 3.5 mm) in a working chamber humidified so as to be a relative humidity of 60% (absolute humidity: 0.012 kg/kgDA) at 25°C using a 20% by mass aqueous ethanol solution as a binder,

Then, the resulting molded article was charged into a baking oven heated to 35°C and was dried for 5 hours. After drying was finished, the temperature was elevated to 70°C over a period of 12 minutes to make the relative humidity in the oven 10% or less. Thereafter, main baking was performed at 380°C over a period of 5 hours to obtain an objective coated catalyst. During the period of the drying step and the baking step of the molded article, air humidified so as to be a relative humidity of 60% (absolute humidity: 0.012 kg/kgDA) at 25°C was continuously allowed to flow into the baking oven.

Composition of the resulting catalyst is as follows:

Mo10V0.8P1.15Cu0.4Cs0.3(NH4)2.3Sb10.

2) Catalytic Oxidation Reaction of Methacrolein

Into a stainless steel reaction tube having an inner diameter of 18.4 mm was packed 10.3 ml of the resulting coated catalyst, and an oxidation reaction of methacrolein was carried out at a raw material gas composition (molar ratio) of methacrolein:oxygen:water vapor:nitrogen = 1:2:4:18.6, a space velocity (SV) of 1,200 hr$^{-1}$, and a reaction bath temperature of 310°C. The reaction was continued at a reaction bath temperature of 310°C for 3 hours at an initial stage of the reaction, then for evaluating thermal stability of the catalyst, the reaction bath temperature was elevated to 350°C, and the reaction was continued for 15 hours (hereinafter, the treatment is referred to as high-temperature reaction treatment). Then, the reaction bath temperature as lowered to 310°C and measurement of reaction performance was conducted. A catalyst resulting in a lower conversion of methacrolein after the high-temperature treatment than the conversion at the initial stage of the reaction tends to show continuous deterioration when industrially used. Results are shown in Table 1.

Example 2

A catalyst was prepared in the same manner as in Example 1 except that 320 g of the granules after the drying step and 22.7 g of antimony trioxide were mixed before pre-baking in Example 1, and the oxidation reaction of methacrolein was carried out. Results are shown in Table 1.

Example 3

To 5680 ml of pure water were added 800 g of molybdenum trioxide, 40.43 g of vanadium pentoxide, and 73.67 g of 85% by mass orthophosphoric acid, and the whole was stirred at 92°C for 3 hours to obtain a reddish brown transparent solution. Then, the solution was cooled to 15 to 20°C and 307.9 of a 9.1% by mass aqueous cesium hydroxide solution and 689.0 g of a 14.3% by mass aqueous ammonium acetate solution were gradually added thereto under stirring and aging was performed at 15 to 20°C for 1 hour to obtain a yellow slurry.

Subsequently, further, 709.9 g of a 6.3% by mass aqueous cupric acetate solution was gradually added to the slurry and aging was further performed at 15 to 20°C for 30 minutes. Then, 32.4 g of antimony trioxide was added to the slurry and aging was performed at 15 to 20°C for 30 minutes. Subsequently, the slurry was spray-dried to obtain granules.

Composition of the obtained granules is as follows:

Mo10V0.8P1.15Cu0.4Cs0.3(NH4)2.3Sb0.4.

Then, 320 g of the granules was baked at 290°C over a period of 5 hours under a stream of air humidified so as to be a relative humidity of 70% (absolute humidity: 0.014 kg/kgDA) at 25°C. There was observed a weight loss of about 4% by mass by the pre-baking. Thereinto was homogeneously mixed 45 g of a strength enhancer (ceramic fibers), and the mixture was coat-molded on 300 g of a spherical porous alumina support (particle size: 3.5 mm) in a working chamber humidified so as to be a relative humidity of 70% (absolute humidity: 0.014 kg/kgDA) at 25°C using a 20% by mass aqueous ethanol solution as a binder.

Then, the resulting molded article was charged into a baking oven heated to 35°C and was dried for 5 hours. After drying was finished, the oven temperature was elevated to 105°C over a period of 20 minutes to make the relative humidity in the oven 10% or less. Thereafter, main baking was performed at 380°C over a period of 5 hours to obtain an objective coated catalyst. During the period of the drying step and the baking step of the molded article, air humidified so as to be a relative humidity of 70% (absolute humidity: 0.014 kg/kgDA) at 25°C was continuously allowed to flow into the baking oven.

Thereafter, an oxidation reaction of methacrolein was carried out in the same manner as in Example 1. Results are shown in Table 1.

Comparative Example 1

A catalyst was prepared in the same manner as in Example 1 except that the absolute humidity of air to be allowed to flow for the pre-baking in Example 1 was changed to 0.006 kg/kgDA, and the oxidation reaction of methacrolein was carried out. Results are shown in Table 1.

Comparative Example 2

A catalyst was prepared in the same manner as in Example 1 except that the absolute humidity of the working chamber for the coat-molding in Example 1 was changed to 0.006 kg/kgDA, and the oxidation reaction of methacrolein was carried out. Results are shown in Table 1.

Comparative Example 3

A catalyst was prepared in the same manner as in Example 1 except that the absolute humidity of air to be allowed to pass for the main baking in Example 1 was changed to 0.006 kg/kgDA, and the oxidation reaction of methacrolein was carried out. Results are shown in Table 1.

[Table 1]

| Results of Methacrylic Acid Oxidation Reaction | | | | | | | |
|---|---|---|---|---|---|---|---|
| | Absolute humidity | | | Oxidation reaction of methacrolein | | | |
| | Introduced gas at pre-baking | Atmosphere of molding chamber | Introduced gas at main baking | | Methacrolein conversion | Methacrylic acid selectivity | Methacrylic acid yield |
| Example 1 | 0.012 | 0.012 | 0.012 | Initial stage of reaction | 82.44 | 79.97 | 65.93 |
| | | | | After high-temperature reaction | 88.25 | 82.32 | 72.65 |
| Example 2 | 0.012 | 0.012 | 0.012 | Initial stage of reaction | 82.66 | 81.61 | 67.46 |
| | | | | After high-temperature reaction treatment | 87.57 | 83.28 | 72.92 |
| Example 3 | 0.014 | 0.014 | 0.014 | Initial stage of reaction | 91.61 | 78.01 | 71.46 |
| | | | | After high-temperature reaction treatment | 92.45 | 80.93 | 74.82 |
| Comparative Example 1 | 0.006 | 0.012 | 0.012 | Initial stage of reaction | 85.09 | 85.33 | 72.61 |
| | | | | After high-temperature reaction treatment | 84.15 | 87.53 | 73.65 |
| Comparative Example 2 | 0.012 | 0.006 | 0.012 | Initial stage of reaction | 89.65 | 83.11 | 74.51 |
| | | | | After high-temperature reaction treatment | 82.35 | 87.48 | 72.04 |

(continued)

| Results of Methacrylic Acid Oxidation Reaction | | | | | | | |
|---|---|---|---|---|---|---|---|
| | Absolute humidity | | | Oxidation reaction of methacrolein | | | |
| | Introduced gas at pre-baking | Atmosphere of molding chamber | Introduced gas at main baking | | Methacrolein conversion | Methacrylic acid selectivity | Methacrylic acid yield |
| Comparative Example 3 | 0.012 | 0.012 | 0.006 | Initial stage of reaction | 87.02 | 82.31 | 71.63 |
| | | | | After high-temperature reaction treatment | 80.97 | 87.24 | 70.63 |
| Units are as follows: absolute humidity: kg/kgDA, conversion/selectivity/yield: % | | | | | | | |

From the above results, it is realized that, when the catalysts containing a hetero polyacid salt prepared by the production process of the present invention as an active component are used, methacrolein conversion is equal to or higher than that at the initial stage of the reaction even after the high-temperature reaction treatment. Namely, when a catalyst containing a hetero polyacid salt prepared by the production process of the present invention as an active component is used, the catalyst has an aptitude as an industrial catalyst and it becomes possible to produce methacrylic acid with stably exhibiting high activity and high performance.

While the invention has been described in detail and with reference to specific embodiments thereof, it will be apparent to one skilled in the art that various changes and modifications can be made therein without departing from the spirit and scope thereof.

The present application is based on Japanese Patent Application No. 2009-271884 filed on November 30, 2009, and the contents are incorporated herein by reference. Also, all the references cited herein are incorporated as a whole.

**Claims**

1.  A process for producing a catalyst comprising, as an active component, a hetero polyacid salt essentially containing molybdenum, phosphorus, vanadium and copper and one or more ingredients selected from alkali metals, alkaline earth metals and ammonia, the process comprising:

    (A) a step of drying a slurry obtained by mixing compound(s) containing these essential ingredients and water, thereby obtaining a dry powder;
    (B) a pre-baking step of baking the dry powder in a baking apparatus into which a gas having a controlled absolute humidity as been introduced, thereby obtaining a catalyst ingredient powder;
    (C) a catalyst molding step of molding the catalyst ingredient powder under an atmosphere gas having a controlled absolute humidity; and
    (D) a baking step of baking the molded catalyst in a baking apparatus into which a gas having a controlled absolute humidity has been introduced;

    the gas to be introduced in the pre-baking step of the above (B) having an absolute humidity of 0.007 to 0.025 kg/kgDA;
    the atmosphere gas in the catalyst molding step of the above (C) having an absolute humidity of 0.007 to 0.025 kg/kgDA; and
    the gas to be introduced in the baking step of the above (D) having an absolute humidity of 0.007 to 0.025 kg/kgDA.

2.  The process according to claim 1, wherein the essential active ingredients of the hetero polyacid further include antimony.

3.  The process according to claim 2, which is a process for producing a catalyst comprising, as an active component, a hetero polyacid salt essentially containing molybdenum, phosphorus, vanadium, copper and antimony and one or more ingredients selected from alkali metals, alkaline earth metals and ammonia, the process comprising:

    (A) a step of drying a slurry obtained by mixing compound(s) containing these essential ingredients except antimony and water, thereby obtaining a dry powder;
    (B) a pre-baking step of baking a mixture of the dry powder and a compound containing antimony in a baking apparatus into which a gas having a controlled absolute humidity has been introduced, thereby obtaining a catalyst ingredient powder;
    (C) a catalyst molding step of molding the catalyst ingredient powder under an atmosphere gas having a controlled absolute humidity; and
    (D) a baking step of baking the molded catalyst in a baking apparatus into which a gas having a controlled absolute humidity has been introduced;

    the gas to be introduced in the pre-baking step of the above (B) having an absolute humidity of 0.007 to 0.025 kg/kgDA;
    the atmosphere gas in the catalyst molding step of the above (C) having an absolute humidity of 0.007 to 0.025 kg/kgDA; and
    the gas to be introduced in the baking step of the above (D) having an absolute humidity of 0.007 to 0.025 kg/kgDA.

4.  The process according to claim 2, which is a process for producing a catalyst comprising, as an active component, a hetero polyacid salt essentially containing molybdenum, phosphorus, vanadium, copper and antimony and one or more ingredients selected from alkali metals, alkaline earth metals and ammonia, the process comprising:

(A) a step of drying a slurry obtained by mixing compound(s) containing these essential ingredients except antimony and water, thereby obtaining a dry powder;

(B) a pre-baking step of baking the dry powder in a baking apparatus into which a gas having a controlled absolute humidity has been introduced, thereby obtaining a catalyst ingredient powder;

(C) a catalyst molding step of molding a mixture of the catalyst ingredient powder and a compound containing antimony under an atmosphere gas having a controlled absolute humidity; and

(D) a baking step of baking the molded catalyst in a baking apparatus in which a gas having a controlled absolute humidity has been introduced;

the gas to be introduced in the pre-baking step of the above (B) having an absolute humidity of 0.007 to 0.025 kg/kgDA;

the atmosphere gas in the catalyst molding step of the above (C) having an absolute humidity of 0.007 to 0.025 kg/kgDA; and

the gas to be introduced in the baking step of the above (D) having an absolute humidity of 0.007 to 0.025 kg/kgDA.

5.   The process according to any one of claims 1 to 4, wherein the gas to be introduced in the pre-baking step of the above (B), the atmosphere gas in the catalyst molding step of the above (C), and the gas to be introduced in the baking step of the above (D) are gases each having a controlled temperature together with a controlled absolute humidity,

the gas to be introduced in the pre-baking step of the above (B) having a temperature of 15 to 90°C and an absolute humidity of 0.007 to 0.025 kg/kgDA;

the atmosphere gas in the catalyst molding step of the above (C) having a temperature of 15 to 35°C and an absolute humidity of 0.007 to 0.025 kg/kgDA; and

the gas to be introduced in the baking step of the above (D) having a temperature of 15 to 90°C and an absolute humidity of 0.007 to 0.025 kg/kgDA.

6.   The process according to any one of claims 1 to 5, wherein the active ingredients of the hetero polyacid salt are molybdenum, phosphorus, vanadium, copper and an alkali metal.

7.   The process according to any one of claims 1 to 5, wherein the active ingredients of the hetero polyacid salt are molybdenum, phosphorus, vanadium, copper, an alkali metal and ammonia.

8.   The process according to any one of claims 1 to 7, wherein the alkali metal is cesium.

9.   The process according to any one of claims 1 to 8, wherein the baking temperature is 200 to 400°C in the pre-baking step of the above (B).

10.   The process according to any one of claims 1 to 9, wherein the molding step of the above (C) is a step of coating an inactive support using a binder to form a coated catalyst.

11.   The process according to claim 10, wherein the binder is water and/or at least one liquid selected from the group consisting of organic compounds each having a boiling point of 150°C or lower at 1 atm.

12.   The process according to any one of claims 1 to 11, wherein the molded article is baked at 100 to 450°C in the baking step of the above (D).

13.   A process for producing methacrylic acid by vapor-phase catalytic oxidation of methacrolein, isobutyraldehyde or isobutyric acid using the catalyst according to any one of claims 1 to 12.

<table>
<tr><td colspan="2">**INTERNATIONAL SEARCH REPORT**</td><td>International application No.<br>PCT/JP2010/071252</td></tr>
</table>

**A. CLASSIFICATION OF SUBJECT MATTER**

*B01J27/24*(2006.01)i, *B01J37/10*(2006.01)i, *C07C51/235*(2006.01)i, *C07C57/055*(2006.01)i, *C07B61/00*(2006.01)n, *C07C51/377*(2006.01)n, *C07C57/05*(2006.01)n

According to International Patent Classification (IPC) or to both national classification and IPC

**B. FIELDS SEARCHED**

Minimum documentation searched (classification system followed by classification symbols)
B01J27/24, B01J37/10, C07C51/235, C07C57/055, C07B61/00, C07C51/377, C07C57/05

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2003-001113 A (Sumitomo Chemical Co., Ltd.),<br>07 January 2003 (07.01.2003),<br>claims; examples; paragraph [0029]<br>(Family: none) | 1-13 |
| A | JP 2003-010691 A (Sumitomo Chemical Co., Ltd.),<br>14 January 2003 (14.01.2003),<br>claims; examples; paragraphs [0011], [0012]<br>(Family: none) | 1-13 |
| A | JP 2008-207127 A (Sumitomo Chemical Co., Ltd.),<br>11 September 2008 (11.09.2008),<br>claims; examples; paragraphs [0014], [0032]<br>(Family: none) | 1-13 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>16 February, 2011 (16.02.11) | Date of mailing of the international search report<br>01 March, 2011 (01.03.11) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2010/071252

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-255689 A (Mitsubishi Rayon Co., Ltd.), 28 September 2006 (28.09.2006), paragraph [0040] (Family: none) | 1-13 |
| A | JP 2005-186065 A (Mitsubishi Chemical Corp.), 14 July 2005 (14.07.2005), claims; examples; paragraphs [0021] to [0023] & US 2005/0159620 A1 & EP 1704918 A1 & WO 2005/053839 A1 & CN 1697695 A | 1-13 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009126943 A
- JP 2036296 B
- JP 2006314923 A

- JP 3797148 B
- JP 2009271884 A